# EUROPEAN PATENT SPECIFICATION

(11) **EP 1 632 204 B1**
(45) Date of publication and mention of the grant of the patent: **18.08.2010**
(21) Application number: 05023924.3
(22) Date of filing: 02.04.2004
(51) Int. Cl.: A61F 9/007, A61M 1/00

(54) **Phacoemulsification needle**
Phakoemulsifikationsnadel
Aiguille de phacoemulsification

(30) Priority: 04.04.2003 US 406572; 23.03.2004 US 806470
(43) Date of publication of application: 08.03.2006
(62) Divisional of application: 04405200.9
(73) Proprietor: Akahoshi, Takayuki, Tokyo 104-0051 (JP); Nallakrishnan, Ravi, Westmont, IL 60559 (US)
(72) Inventor: Akahoshi, Takayuki, Tokyo 104-0051 (JP)
(74) Representative: Liebetanz, Michael

(56) References cited:
- WO-A-96/38091
- US-A- 4 767 404
- US-A- 6 126 629
- US-A1- 2002 099 325
- US-B1- 6 491 670

## Description

### Field of the invention

This invention relates to a phacoemulsification needle for an ultrasonic surgical instrument, the needle being designed for promoting cavitation in eye tissue and for the removal of fragmented lens from the eye.

### Background of the invention

Phacoemulsification has become the preferred form of cataract, i.e. a cloudy eye lens, removal. One of the main advantages of phacoemulsification is, that only a small incision into the cornea or sclera of an eye is needed to remove the cataract. Furthermore, the removal of the cataract can be done very quickly. After the cataract is removed, an intraocular lens is inserted to replace the original lens.

The phacoemulsification technique uses a hand held microsurgical tool known as phacoemulsifier. This phacoemulsifier comprises a handpiece and a small diameter needle with a tip to be inserted into the small incision of the eye. The needle and therefore the tip are vibrated by an ultrasonic source. It breaks the cataract into small fragments and pieces, which are sucked out through the same tip in a controlled manner. The tip is therefore designed for emulsifying, fragmenting and/or cutting tissue and also comprises a central hollow bore or lumen for the suction or aspiration of the fragments.

During the procedure, an irrigation solution is introduced to maintain the pressure and to prevent the eye from collapsing. In order to introduce the irrigation solution, the needle is usually covered by a sleeve and the solution flows via the space between this sleeve and the needle. The solution is therefore also used to cool the tip, which is heating up during the phacoemulsification.

Usually, the needle and often the handpiece must be changed after the removal of the cataract nucleus in order to remove the residual cortex. It takes time to change the handpiece and the continuation of the surgery is disrupted.

US-A-5'653'724 discloses a phacoemulsification needle which is angled to provide more comfortable ergonomic angle during phacoemulsification and lens cortex removal. This angled needle is also considered to produce less heat when emulsifying the lens. Another angled phacoemulsification needle with a concentric sleeve is disclosed in US-5'993'409.

Different shapes of phacoemulsification needles with slits, a second infusion hole and/or with increased outside diameter at the distal end of the needle body and the needles being surrounded by sleeves are described in US-A-5'989'209, US-A-6'159'175, EP-A-1'103'238, WO 00/74615 and US 2002/0099325.

Other techniques for cataract removal use laser energy to remove the cataract. A laser/aspiration probe is used for breaking and removing the lens. A separate infusion or irrigation probe is used for the irrigation solution.

### Summary of the invention

It is an object of the invention to provide a phacoemulsification needle which can be used for the removal of the cataract nucleus as well as the residual cortex.

This object is achieved with a phacoemulsification needle according to claim 1.

With a ball-shaped or curved tip design and at least two openings being arranged in the region of the distal end of the tip, it is possible to remove both the cataract nucleus and the residual cortex.

This tip can be used for irrigation and aspiration without having to change the tip and therefore having the advantage that time can be saved. The tip can be used for irrigation and/or aspiration if desired. It can not only be used for phacoemulsification, but also for cortical removal and for polishing the capsule.

The needle according to the invention can be used with or without an irrigation sleeve.

In preferred embodiments, the tip has a ball-shaped, round, spherical, oval or smooth continuous curved shape.

The tip has at its distal end rounded edges. This avoids unwished damage of tissue. Due to the inventive shape of the tip, especially its broad and smooth spherical surface area of the tip, no damage to the posterior capsule can occur.

In a preferred embodiment, the tip comprises a slit extending in longitudinal direction of the needle. When a high vacuum setting is used with the phacoemulsifier, the anterior chamber, formed of a part of the lumen extending in the tip, becomes unstable when occlusion break occurs. In this case, small amount of irrigation fluid will continue to flow through the aspiration lumen. Furthermore, when occlusion surge occurs, the amount of surge can be reduced. Instead of the slit, an opening can be provided in a region adjacent to the tip.

The tip can comprise more than one opening of the lumen, these openings being arranged in the region of the distal end. In this case, the distal end can have instead of a flat shape a rounded one. This embodiment even increases the safety from rupturing the posterior capsule.

Further preferred embodiments of the invention are described in the dependent claims.

### Brief description of the drawings

The present invention will be more clearly understood with reference to the following detailed description of preferred embodiments, taken in conjunction with the accompanying drawings, in which
- Figure 1: shows a perspective view of a needle according to a first embodiment;
- Figure 2: shows a side view of the needle according to figure 1;
- Figure 3: shows a longitudinal view of the needle according to figure 1;
- Figure 4: shows a magnified part of the needle tip according to figure 3;
- Figure 5: shows a front view of the needle according to figure 1;
- Figure 6: shows a front view of a needle according to a second embodiment;
- Figure 7: shows a perspective view of a needle according to a third embodiment;
- Figure 8: shows a side view of the needle according to figure 7;
- Figure 9: shows a longitudinal view of the needle according to figure 7;
- Figure 10: shows a magnified part of the needle tip according to figure 7;
- Figure 11: shows a front view of the needle according to figure 7;
- Figure 12: shows a side view perspective view of a needle according to a fourth embodiment;
- Figure 13: shows a longitudinal view of the needle according to figure 12;
- Figure 14: shows a magnified part of the needle tip according to figure 13;
- Figure 15: shows a front view of the needle according to figure 12;
- Figure 16: shows a side view of a needle according to a fifth embodiment;
- Figure 17: shows a longitudinal of the needle according to figure 16;
- Figure 18: shows a magnified part of the needle tip according to figure 17;
- Figure 19: shows a longitudinal view of a needle according to the invention in a sixth embodiment;
- Figure 20: shows a perspective view of the needle according to figure 19 and
- Figure 21: shows a front view of the needle according to figure 19.

### Description of the preferred embodiments

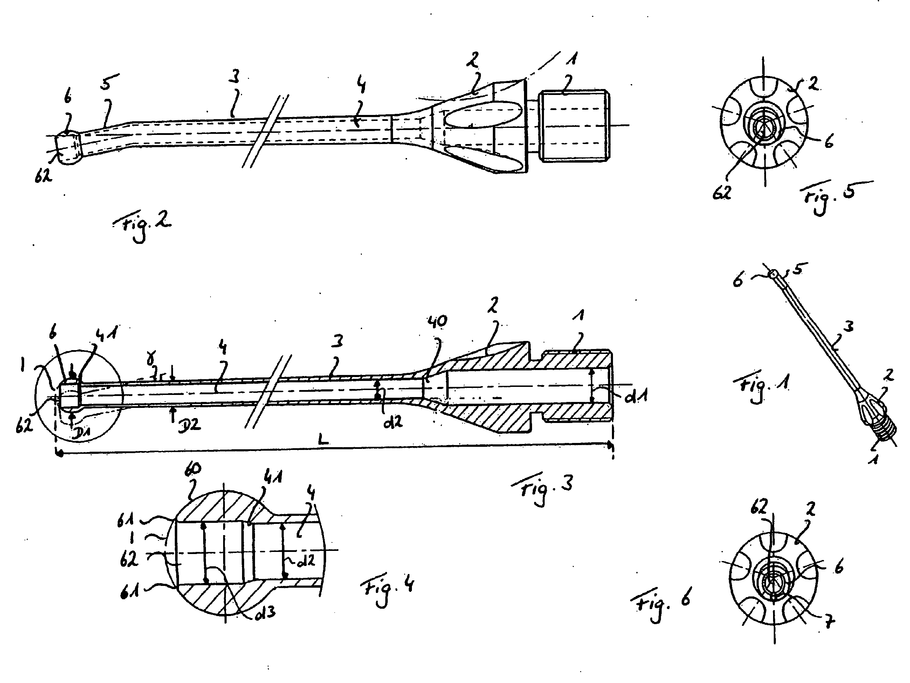

With reference to figures 1 and 2, there is shown a phacoemulsification needle which is not part of the present invention. The needle may be formed from any conventional material as is known in the art for the manufacture of phacoemulsification needles. Usually, it is made of titan.

The needle comprises, starting with its proximal end and ending with its distal end, a threaded portion 1, a hub 2, a shaft 3 and a tip 6 having at least one opening. The threaded portion 1 and the hub 2 are used to fix the needle on a ultrasonic device of the phacoemulsification handpiece (not shown) in order to couple ultrasonic energy to the needle.

The needle further comprises, with reference to figures 3 and 4, an aspiration lumen 4 extending through the whole longitudinal length of the needle shaft 3 and communicating with the opening of the tip for aspiration of irrigation fluid and therefore for removal of fragments and pieces of the cataract nucleus and residual cortex. Preferably, the lumen 4 comprises a first step 40 in the region of the hub 2 neighboring the shaft 3. The first diameter d1 of the part of the lumen extending within the hub 2 is therefore larger than the second diameter d2 of the lumen part extending within the shaft 2. This first step 40 has preferably a conical shape.

A second step 41, which has preferably also a conical shape, is located within the tip 6, wherein the second diameter d2 is smaller than a third diameter d3 of the lumen part extending within the shaft 2. This diameter d3 corresponds with the diameter of the opening of the tip 6.

With exception of the steps 40, 41, the lumen 4 preferably has throughout its whole length the same size. In particular the lumen part extending in the tip 6 has a cylindrical shape.

The shaft 3 is preferably angled. However, it is also possible to have a rectilinear shaft. In the here described embodiment however, the shaft 3 comprises an angled portion 5 neighboring the tip 6. The aspiration lumen 4 within the tip extends in a rectilinear way.

The tip 6 as shown in figure 5 as well and which is arranged at the distal end of the shaft 3, has a larger outer diameter D1 than the outer diameter D2 of the shaft 3. Furthermore, it has a ball-shaped surface 60 and a flat distal end 62. In this preferred embodiment, the flat distal end 62 is formed by the opening. However, the opening can also be smaller, so that the end face of the tip 6 is flat. In the here described embodiment however, the end face is formed by rounded blunt edges 61. The above mentioned second step 41 is preferably located in the tip 6 itself at a distance to the transition of the ball-shaped surface 60 to the surface of the shaft 3. In figure 4, a curved line 1 is shown at the distal end of the tip 6. This line 1 is just an auxiliary line showing that, with exception of the flat distal end, the tip has a completely spherical surface.

A preferred embodiment of the needle has an outer diameter D2 for the shaft 2 of approximately 1.06 mm, an outer diameter D1 of the tip 6 of approximately 1.6 mm, a first diameter d1 of the lumen 4 of approximately 1.32 mm, a second diameter d2 of approximately 0.72 mm, a third diameter d3 of approximately 0.8 mm, a length L of the tip 5 (extending up to the curved line 1) of approximately 1.46 mm and a distance X from the curved line 1 to the second step 41 of approximately 1.05 mm. The radius of curvature of the rounded edges 61 is approximately 0.15 mm, the angle of the first step 40 approximately 34° and the angle of the second step 41 approximately 30°. The angle γ between shaft 3 and tip 4 lays between 10 and 15°. Here it is approximately 15°. The total length of the needle up to the curved line 1 is approximately 30.15 mm.

Figure 6 shows a second embodiment of the needle which is not part of the invention. The needle is built in the same way as the needle according to the previously described figures. The only difference is, that this tip 6 comprises a slit 7 extending in longitudinal direction of the needle, which communicates with the aspiration lumen 4.

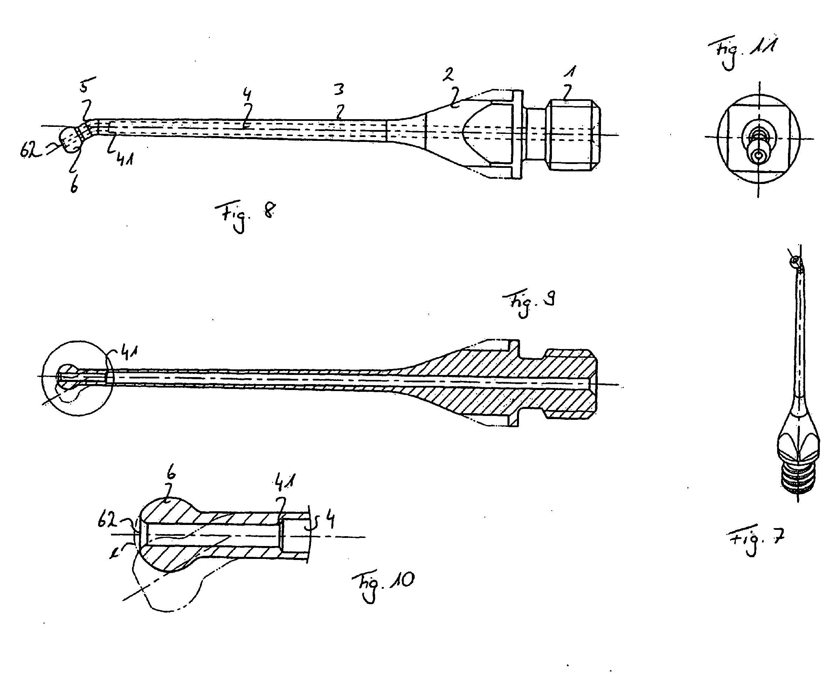

Figures 7 to 11 show a third embodiment of the needle which is not part of the invention. The threaded portion 1 is slightly shorter than the one shown in figure 2. The hub 2 has not a generally circular cross section but a rectangular, preferably a quadratic one. This hub 2 and this threaded portion 1 can also be used with the tips disclosed in the other embodiments and the hup and threaded portion disclosed in figure 2 can be used with this tip.

The aspiration lumen 4 extending through the needle shaft 3 comprises a second step 41 like the one shown in figure 1. However, this second step 41 is arranged is located outside of the tip 6. This second step 41 is in this case preferably arranged behind the angled portion 5. If the shaft 3 is rectilinear, the second step 41 is preferably arranged in a distance behind the tip 6 which has approximately the same length as the tip 6. This step 41 reduces the cross section of the lumen 4. In addition, no first step is located within the hub 2.

This second step 41 however can also be arranged in the tip 6. Furthermore, instead of this step, the second step according to figure 2 can be used. The embodiment according to figure 2 can also have the second step 41 being arranged in the shaft 3 instead in the tip 6, wherein the second step 41 can reduce or increase the cross section of the opening at the distal end 62. Furthermore, this second step can, but must not be conical.

Since the opening is at the distal end 62 according to this third embodiment, the tip 6 has a curved shape, which is slightly different to the one of the first embodiment. The distal end 62 is less flat and the curved line 1 is shorter.

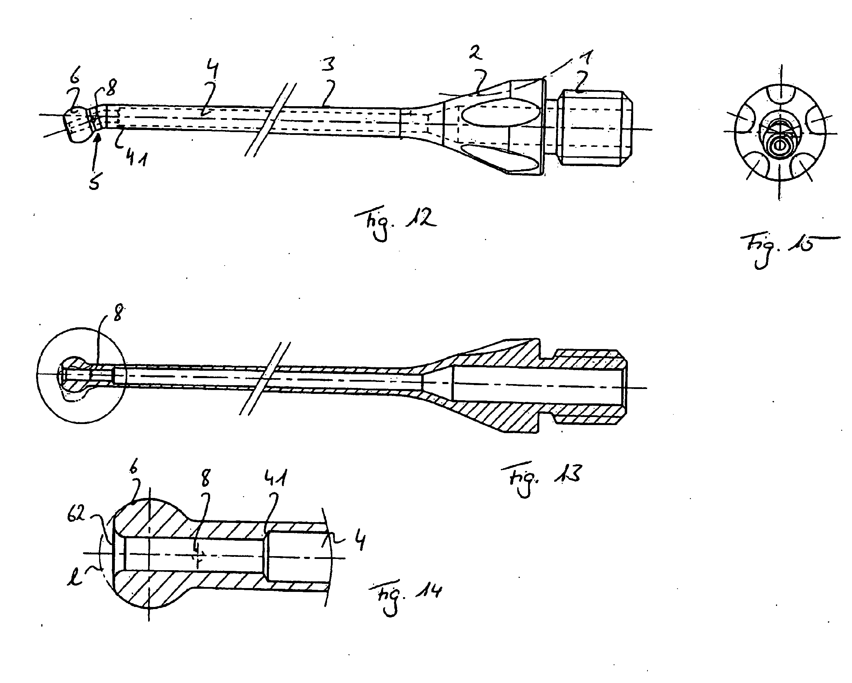

Figures 12 to 15 show a fourth embodiment which is not part of the invention. In this embodiment, an opening 8 in the shape of a circular hole is located behind the tip 6. If the shaft 3 is angled, this opening 8 is preferably located in the angled portion 5. This opening 8 communicates with the aspiration lumen 4. Like the slit 7, this opening 8 helps when the anterior chamber, formed of a part of the lumen extending in the tip, becomes unstable when occlusion break occurs. In this case, small amount of irrigation fluid will continue to flow through the aspiration lumen. Furthermore, when occlusion surge occurs, the amount of surge can be reduced by this slit 7 or this opening 8. This fourth embodiment can also comprise instead of the opening 8 the slit 7 shown in figure 6.

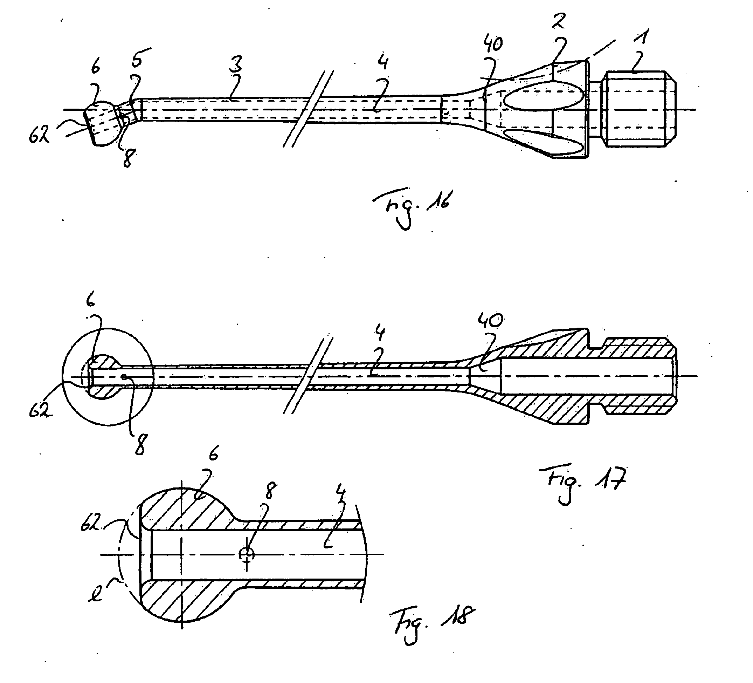

Figures 16 to 18 show a fifth embodiment which is not part of the invention. Here, there exists no second step 41, neither in the shaft 3 nor in the tip 6. This embodiment can be used with the opening 8 or the slit 7 or without any of them. Furthermore, any of the above mentioned embodiments can comprise the opening 8 as well as the slit 7.

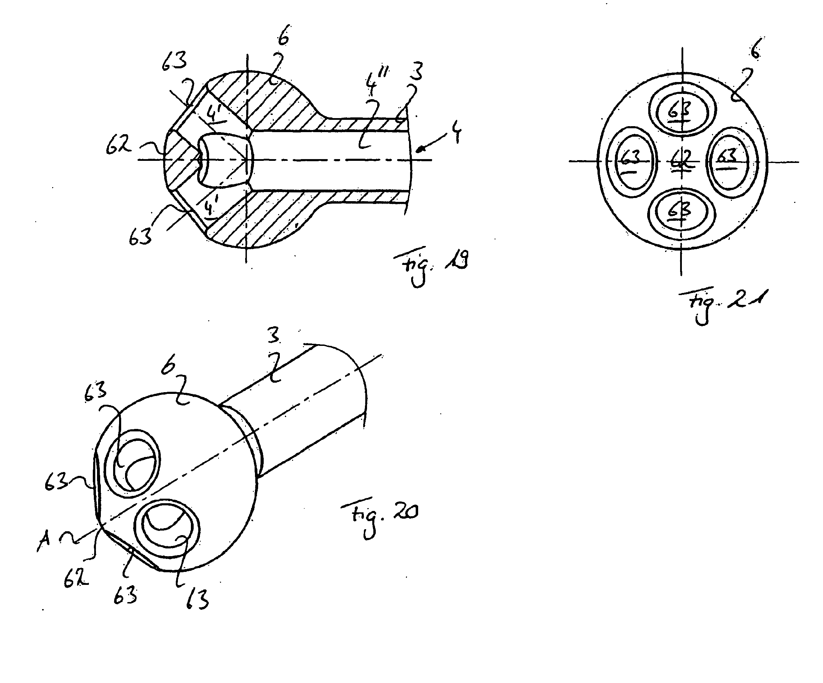

An embodiment according to the present invention is shown in the figures 19 to 21. The first and second steps 40 and 41 can be arranged or being nonexistent as described in any of the previous embodiments. Furthermore, this embodiment can comprise an opening 8 and/or a slit 7 or none of them. The tip 6 of this embodiment still has a curved shaped. This embodiment however comprises instead of the flat distal end 62 being defined by a distal opening of the lumen 4 at least two openings 63 of the lumen 4 being arranged in the region of the distal end 62. Here, we have such four openings 63. However, there can also be three, five or more openings. Preferably, those are arranged in a symmetrical manner around the longitudinal axis A defined by the shaft 3. These openings 63 have like the single opening of the other embodiments blunt or rounded edges 61. In order to communicate with these openings 63, the lumen 4 is divided into sublumens 4', each sublumen 4' ending in one of the mentioned openings 63. This division is preferably made only in the tip 6. If a slit 7 or an opening 8 are provided, they can be arranged in the region of the undivided lumen 4 " or the different sublumens 4' can comprise a subslit each ending in a common slit 7 or each sublumen 4" can comprise an one opening 8.

In the above description, the shape of the tip was normally described a ball-shaped. However, the tip 6 can have a generally curved shape, preferably a ball-shaped, round, spherical, oval or smooth continuous curved shape.

The needle according to the invention can be used for the ultrasonic part of the phacoemulsification procedure, i.e. for the removal of the cataract nucleus, as well as for the irrigation/aspiration part of the procedure, i.e. for the removal of the residual tissue.

### List of reference numbers

- 1: threaded portion
- 2: hub
- 3: shaft
- 4: lumen
- 4': sublumen
- 4'': undivided lumen
- 40: first step
- 41: second step
- 5: angled portion
- 6: tip
- 60: ball-shaped surface
- 61: rounded edges
- 62: flat distal end
- 63: opening of the lumen
- 7: slit
- 8: opening
- d1: first diameter
- d2: second diameter
- d3: third diameter
- D2: outside diameter of the shaft
- D1: outside diameter of the tip
- L: length of the tip
- 1: curved line
- X: distance
- γ: angle
- A: longitudinal axis

## Claims

1. A phacoemulsification needle comprising
a shaft (3),
a tip (6), disposed at a distal end of said shaft (3), said tip (6) having a larger outer diameter than said shaft (3), and
an aspiration lumen (4) extending through said shaft (3) and said tip (6),
wherein said tip (6) has a curved shape and wherein said tip (6) has at least two openings (63) communicating with said aspiration lumen (4) and being arranged in the region of a distal end (62) of said tip (6),
**characterized in that** said at least two openings (63) have rounded edges.

2. The needle according to claim 1, wherein the tip (6) has four openings (63).

3. The needle according to claim 1, wherein the distal end (62) has a curved surface.

4. The needle according to claim 1, wherein said shaft (3) defines a longitudinal axis (A) and wherein the at least two openings are arranged in a symmetrical manner around said longitudinal axis (A).

5. The needle according to claim 1, wherein said aspiration lumen (4) comprises a step (41) within the tip (6).

6. The needle according to claim 1, wherein said aspiration lumen (4) comprises a step (42) within the shaft (3).

7. The needle according to claim 1, wherein said tip (6) comprises a slit (7) extending in a longitudinal direction of the needle and communicating with said aspiration lumen (4).

8. The needle according to claim 1, wherein said shaft (3) comprises an opening extending through its surface, the opening communicating with said aspiration lumen (4).

9. The needle according to claim 1, wherein said aspiration lumen (4) is divided into sublumens (4'), each sublumen (4') ending in one of said openings (63).

10. The needle according to claim 9, wherein said aspiration lumen (4) is divided from an undivided lumen (4") into said sublumens (4') within said tip (6).

## Patentansprüche

1. Phakoemulsifikationsnadel, aufweisend
einen Schaft (3),
eine an einem distalen Ende des Schafts (3) angeordnete Spitze (6), die einen grösseren Aussendurchmesser als der Schaft (3) aufweist, und
ein Aspirationslumen (4), welches sich durch den Schaft (3) und die Spitze (6) hindurch erstreckt,
wobei die Spitze (6) eine gewölbte Form aufweist und wobei die Spitze (6) mindestens zwei Öffnungen (63) aufweist, die mit dem Aspirationslumen (4) kommunizieren und im Bereich eines distalen Endes (62) der Spitze (6) angeordnet sind,
**dadurch gekennzeichnet, dass** die mindestens zwei Öffnungen (63) abgerundete Kanten aufweisen.

2. Nadel nach Anspruch 1, wobei die Spitze (6) vier Öffnungen (63) aufweist.

3. Nadel nach Anspruch 1, wobei das distale Ende (62) eine gewölbte Oberfläche aufweist.

4. Nadel nach Anspruch 1, wobei der Schaft (3) eine Längsachse (A) definiert und wobei die mindestens zwei Öffnungen in symmetrischer Weise um die Längsachse (A) herum angeordnet sind.

5. Nadel nach Anspruch 1, wobei das Aspirationslumen (4) eine Stufe (41) innerhalb der Spitze (6) aufweist.

6. Nadel nach Anspruch 1, wobei das Aspirationslumen (4) eine Stufe (42) innerhalb des Schafts (3) aufweist.

7. Nadel nach Anspruch 1, wobei die Spitze (6) einen Schlitz (7) aufweist, der sich in einer Längsrichtung der Nadel erstreckt und mit dem Aspirationslumen (4) kommuniziert.

8. Nadel nach Anspruch 1, wobei der Schaft (3) eine sich durch seine Oberfläche erstreckende Öffnung aufweist, und wobei die Öffnung mit dem Aspirationslumen (4) kommuniziert.

9. Nadel nach Anspruch 1, wobei das Aspirationslumen (4) in Sublumina (4') aufgeteilt ist, und wobei jedes Sublumen (4') in einer der Öffnungen (63) endet.

10. Nadel nach Anspruch 9, wobei das Aspirationslumen (4) innerhalb der Spitze aus einem ungeteilten Lumen (4") in die Sublumina (4') aufgeteilt ist.

## Revendications

1. Aiguille de phacoémulsification, comprenant:
une tige (3),
une pointe (6), située à une extrémité distale de ladite tige (3), ladite pointe (6) présentant un diamètre extérieur supérieur à celui de ladite tige (3), et
une lumière d'aspiration (4) qui s'étend à travers ladite tige (3) et ladite pointe (6),
dans laquelle ladite pointe (6) présente une forme courbe, et dans laquelle ladite pointe (6) comporte au moins deux ouvertures (63) qui communiquent avec ladite lumière d'aspiration (4) et qui sont formées dans la région d'une extrémité distale (62) de ladite pointe (6),
**caractérisée en ce que** lesdites au moins deux ouvertures (63) présentent des bords arrondis.

2. Aiguille selon la revendication 1, dans laquelle la pointe (6) comporte quatre ouvertures (63).

3. Aiguille selon la revendication 1, dans laquelle l'extrémité distale (62) présente une surface courbe.

4. Aiguille selon la revendication 1, dans laquelle ladite tige (3) définit un axe longitudinal (A), et dans laquelle lesdites au moins deux ouvertures sont disposées de façon symétrique autour dudit axe longitudinal (A).

5. Aiguille selon la revendication 1, dans laquelle ladite lumière d'aspiration (4) comporte un gradin (41) à l'intérieur de la pointe (6).

6. Aiguille selon la revendication 1, dans laquelle ladite lumière d'aspiration (4) comporte un gradin (42) à l'intérieur de la tige (3).

7. Aiguille selon la revendication 1, dans laquelle ladite pointe (6) comporte une fente (7) qui s'étend dans une direction longitudinale de l'aiguille et qui communique avec ladite lumière d'aspiration (4).

8. Aiguille selon la revendication 1, dans laquelle ladite tige (3) comporte une ouverture qui s'étend à travers sa surface, l'ouverture communiquant avec ladite lumière d'aspiration (4).

9. Aiguille selon la revendication 1, dans laquelle ladite lumière d'aspiration (4) est divisée en sous-lumières (4'), chaque sous-lumière (4') se terminant dans l'une desdites ouvertures (63).

10. Aiguille selon la revendication 9, dans laquelle ladite lumière d'aspiration (4) est divisée à partir d'une lumière non divisée (4") en lesdites sous-lumières (4') à l'intérieur de ladite pointe (6).
